# EUROPEAN PATENT APPLICATION

(11) **EP 4 154 968 A1**
(43) Date of publication of application: **29.03.2023**
(21) Application number: 21736622.8
(22) Date of filing: 21.05.2021
(51) Int. Cl.: B01D 53/14, B01D 53/62, B01D 53/73, B01D 53/78, B01D 53/84, C12M 1/107, C12M 1/00

(54) **PROCESS FOR CAPTURING CARBON DIOXIDE FROM A GAS**

(30) Priority: 22.05.2020 WO PCT/ES2020/070339
(71) Applicant: TROVANT TECHNOLOGY, S.L, 47016 Valladolid (ES)
(72) Inventor: COLZI LOPES, Alexandre, 47016 Valladolid (ES); ESTRADA PEREZ, Jose Manuel, 47016 Valencia (ES)
(74) Representative: Pons
(86) International application number: PCT/ES2021/070366
(87) International publication number: WO 2021/234207

(57) **Abstract**

The invention relates to a process for capturing carbon dioxide in a gas (3), intended to be carried out in two reactors (1,2), which comprises introducing an alkaline solution (4) into the first reactor (1) and bubbling the gas (3) such that the carbon dioxide is absorbed in the form of carbonates in said alkaline solution (4) forming a first liquid (6), and consequently a methane gas stream (5) is formed in an upper portion of the first reactor (1), feeding the first liquid (6) to the second reactor (2) which in turn comprises chemoautotrophic nitrifying microorganisms (7), and introducing an air stream (9) into the reactor (2), such that the autotrophic microorganisms (7) use the carbonates of the first liquid (6) to grow, thus generating solid biomass (12) within a resulting liquid (11) in the second reactor (2).

## Description

### OBJECT OF THE INVENTION

The present invention relates to a process for capturing carbon dioxide. More particularly, the present invention is included in the field of processes for improving a gas, and more specifically, in the processes for capturing CO₂ by means of nitrogen microorganisms. An object of the present invention is capturing carbon dioxide starting from a gas, which may preferably contain methane, such as a biogas or gases derived from oil, that by using chemoautotrophic nitrifying microorganisms, grow and generate biomass in the process, wherein said biomass in turn can be reused in an anaerobic digestion process in order to generate the starting biogas.

### BACKGROUND OF THE INVENTION

The gaseous mixtures of methane and CO₂ that can be obtained from organic waste (urban solid waste, agri-food waste, sludge and/or wastewater) for the subsequent use thereof as vehicular fuel and injection into natural gas networks (among others) in the form of methane, need CO₂ to be removed. Currently, there are several known processes for said removal/separation of that CO₂ from the gas stream, mostly based on physicochemical processes: pressure swing adsorption (PSA), water scrubbers, organic solvent amines and membrane processes.

Likewise, some solutions based on capturing CO₂ by means of photosynthetic biomass are known in the state of the art such as, for example, photoautotrophic microorganisms (microalgae, purple bacteria or others) which use it to grow. However, such processes require large surfaces in order to collect sunlight.

Additionally, the known solutions require pressures well above atmospheric pressure and do not take advantage of the waste in the carbon dioxide removal/separation step to be used in the biogas generation steps, being significantly energy-intensive and wasting the resources used, as well as in the treatment of waste or residues. Therefore, they are solutions that are not very ecological.

### DESCRIPTION OF THE INVENTION

The present invention aims to solve some of the problems mentioned in the state of the art. More specifically, the present invention describes the process for capturing carbon dioxide from inside a gas, intended to be carried out in at least two reactors, wherein said process comprises, at least, the steps of:
a.) Introducing an alkaline solution into the first reactor and bubbling or purifying the gas such that the carbon dioxide is absorbed in the form of carbonates in said alkaline solution, forming a first liquid comprising solubilised carbonates, and therefore a clean gas stream is formed in an upper portion of the first reactor,
b.) feeding the first liquid to the second reactor, which in turn comprises chemoautotrophic nitrifying microorganisms,
c.) introducing an air stream into the second reactor and a stream of reduced nitrogen nutrients, such that the chemoautotrophic nitrifying microorganisms use the solubilised carbonates of the first liquid to grow, thus generating solid biomass inside a resulting liquid in the second reactor.

In a preferred embodiment, in the first reactor, a ratio of gas and alkaline solution from 0.3 to 4 can be used.

Likewise, pH values from 6 to 12 can be used in the first reactor.

With variable residence times between 12 min and 50 min, CO₂ removals from 55% to 100% have been achieved.

Likewise, in the second reactor a ratio between the first liquid and the reduced nitrogen nutrients -such as ammonia- from 5.1 to 11.5 can be used.

In a preferred embodiment, residence times of the first liquid in the second reactor from a plurality of minutes to 7 days can be used, resulting in an ammonia removal from 25% to 100%.

Advantageously, the residence time of the first liquid in the second reactor can be from 30 minutes to 1 hour.

Alternatively, the residence time of the first liquid in the second reactor can be from 30 minutes to 3 hours.

The chemoautotrophic nitrifying microorganisms can be added in the second reactor initially -after or before the inlet of the first liquid- such that said microorganisms do not have to be added continuously.

Preferably, the reactor is initially inoculated with said nitrifying microorganisms and then the chemoautotrophic microorganism culture can grow and is kept in the second reactor.

The starting gas comprising carbon dioxide can be one that comes from combustion gases, gases derived from oil drilling or, alternatively, a biogas.

Advantageously, the gas can be a biogas of the type produced in an anaerobic digestion process, for which reason, therefore, a portion of the biomass generated in the second reactor can be reused in the anaerobic digestion process in order to increase the generation of the starting biogas, the object of the CO₂ capture.

Likewise, in a preferred embodiment waste nutrients resulting from the anaerobic digestion can be used which act as reduced nitrogen nutrients in the second reactor.

In a preferred embodiment, the resulting clean gas in the upper portion of the second reactor is methane, more specifically when it is a starting gas like biogas, natural gas or gases derived from the oil drilling that comprise methane.

Advantageously, in an additional step the resulting liquid after the biomass generation is recirculated to the first reactor acting as an alkaline solution, thus improving the overall efficiency of resources of the process.

Likewise, the biomass generated in the second reactor can be recirculated out of the second reactor and return, for regulation purposes.

The process may have an intermediate step wherein the resulting liquid which is recirculated to the first reactor is previously adjusted in pH by means of a pH adjustment device. Said device can be located inside the first reactor or in an intermediate location between the first reactor and the second reactor.

The process may have an additional step wherein one or more nutrients are introduced into the second reactor, in order to facilitate the growth of biomass starting from the carbonates solubilised in the first liquid. Said nutrients can be selected from a list of at least one, from among: ammonium, phosphorus, fertilisers, slurries, ammonium salts or urea.

Preferably, the one or more nutrients comprise, at least, ammonium.

All the steps mentioned above can be performed between 0 manometric bar and 6 manometric bar, without needing high pressures to capture the CO₂.

Advantageously, the liquid column of the second reactor may not be pressurised.

Optionally, the process for capturing carbon dioxide from a gas is intended to be carried out in three reactors, wherein in step a.) the gas is purified and wherein said process further comprises the step of:
d.) feeding the first liquid comprising solubilised carbonates to a third reactor wherein the carbon dioxide absorbed in step a.) is partially or totally desorbed, regenerating the absorbent capacity thereof, wherein step d.) takes place between steps a.) and b.) and before step c.).

With variable residence times between 20 s and 120 s for the purification of the gas in the third reactor, CO₂ removals from 55% to 100% have been achieved.

Preferably, prior to step d.), the following is carried out:
e.) a step of flashing the first liquid wherein the pressure thereof is reduced, and optionally:
f.) a step of recirculating a stream rich in CH₄ to the inlet to the first reactor, mixing it with the gas. Optionally, after step e.), step d.) is carried out.

Preferably, the desorption of step d.) takes place at a temperature between 90 and 130°C.

Optionally, after step d.) and before step b.) the process comprises:
f.) a cooling step wherein the water evaporated in step d.) is recovered.

Optionally, after step c.) the process comprises:
g.) a step of compensating for a stream purged in step c.), and
h.) a step of feeding said current compensated for in step g.) before step a.).

All the steps from step a.) to step h.) can be performed between 2 manometric bar and 15 manometric bar, without needing high pressures to capture the CO₂.

Note that the process has the absorption and subsequent capture of CO₂ by means of autotrophic nitrifying organisms which in turn generate biomass starting from inorganic carbon, without needing sunlight and the activity thereof is not based on photosynthesis.

### DESCRIPTION OF THE DRAWINGS

To complement the description that is being made and for the purpose of helping to better understand the features of the invention, according to a preferred practical embodiment thereof, a set of drawings is attached as an integral part of said description, a set of drawings wherein the following has been depicted, with an illustrative and non-limiting character:
Figure 1 shows a schematic view of a preferred embodiment of the process for capturing carbon dioxide from within a gas.
Figure 2 shows a schematic view of a preferred embodiment of the process for capturing carbon dioxide from within a biogas coming from an anaerobic digestion.
Figure 3 shows a schematic view of a preferred embodiment of the process when there is an intermediate desorption step.

### PREFERRED EMBODIMENT OF THE INVENTION

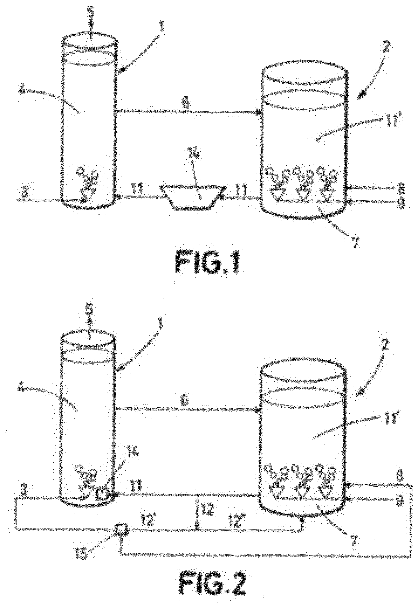

Figure 1 shows a schematic view of a preferred embodiment of the process for capturing carbon dioxide from inside a gas. More particularly, figure 1 shows that said is intended to be carried out in two reactors (1,2), and it has a first step comprising bubbling the gas in the first reactor (1) which in turn comprises an alkaline solution (4), such that the carbon dioxide is absorbed in the form of carbonates in said alkaline solution (4) forming a first liquid (6), and consequently a clean gas stream (5) is formed in an upper portion of the first reactor (1). Said clean gas (5) can be methane in a preferred embodiment wherein the starting gas (3) is a biogas, or gases derived from petroleum, not being the case in, for example, combustion gases.

Subsequently, as shown in Figure 1, the process comprises a successive step to feed the first liquid (6) to the second reactor (2), which in turn comprises chemoautotrophic nitrifying microorganisms (7), wherein said second reactor (2) further comprises an air inlet (9) and a source of reduced nitrogen nutrients (8) which in the described preferred embodiment comprise, at least, ammonium.

Consequently, the chemoautotrophic microorganisms (7) use the carbonates of the first liquid (6) to grow, thus generating solid biomass (12) within a resulting liquid (11) contained in the second reactor (2).

In the preferred embodiment described by figure 1, the process comprises, furthermore, recirculating the resulting liquid (11) to the first reactor (1) acting, as well as the alkaline solution (4) necessary to solubilise the carbon dioxide in the form of carbonates.

In an alternative embodiment, the biomass (12) can be recirculated out of the reactor and later return with the object of maintaining the nitrifying biomass constant.

In the preferred embodiment described by figure 1, the resulting liquid (11) is adjusted in pH by means of the device (14) before being introduced into the first reactor (1) and located between the first reactor (1) and the second reactor (2).

In an alternative embodiment, the biomass (12) is recirculated out of the second reactor (2) through the device (14) before returning to said second reactor (2).

Figure 2 shows a schematic view of a preferred embodiment of the process for capturing carbon dioxide from within a biogas (3') coming from an anaerobic digestion (15).

More particularly, Figure 2 shows that the process in a second preferred embodiment comprises reusing a portion (12') of the biomass (12) generated in the second reactor (2) in the anaerobic digestion (15) in order to in turn produce the starting biogas, improving the overall efficiency of both processes in synergy.

The biomass (12) generated in the second reactor (2) increases the production of biogas and prevents the need to manage waste and sludge from said excess biomass (2).

Likewise, in the second preferred embodiment described, the process further comprises using waste nutrients (8) resulting from anaerobic digestion (15) and introducing said waste nutrients (8) into the second reactor, thus acting as the source of reduced nitrogen nutrients (8) and further taking advantage of the overall efficiency in the resources used in both processes.

In the preferred embodiment described by figure 2, the resulting liquid (11) is adjusted in pH by means of the device (14) which is in turn located inside the first reactor (1).

Lastly, according to the described preferred embodiment, a second portion of biomass (12") that is not used in the anaerobic digestion process (14) is reintroduced into the reactor (2).

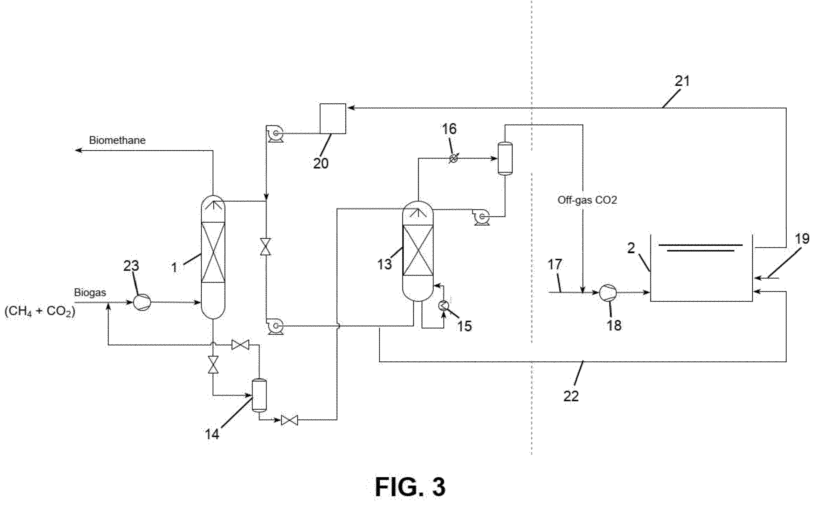

According to the embodiment shown in figure 3, the biogas (CH₄ + CO₂) enters the system, is compressed in a first compressor (23) and enters the first reactor (1) or scrubber-type absorption column through the lower portion. In this first reactor (1), the gas rises and comes into contact with an alkaline liquid solution containing carbonates (sodium or potassium carbonate solution). As it rises through the first reactor (1), the CO₂ of the gas is transferred to the liquid solution, obtaining in the head of the first reactor (1) a stream of purified CH₄ (biomethane). The key operating parameters of this unit are the operating pressure (range of 2 to 15 bar), gas and liquid velocity and gas/liquid ratio.

The liquid rich in CO₂ collected at the bottom of the first reactor (1) is directed to an optional flashing unit (14) wherein the pressure is reduced in order to subsequently desorb the methane CH₄ which may have stayed absorbed in the liquid. That stream of gas rich in CH₄ can be recirculated from the head of the flashing unit (14) to the inlet of the system and is mixed with the fresh biogas, minimising the losses of CH₄.

The liquid stream rich in CO₂ obtained in the bottom of the flashing unit (14) is directed to the head of a third reactor (13) or desorption column. In this third reactor or desorption column, the liquid trickles from the upper portion and at the bottom, temperature is applied by means of a heat exchanger (15) or electrical resistor in order to increase the temperature of the liquid. In this manner, the absorbed CO₂ in the alkaline liquid carbonate solution in the first reactor (1) is partially or totally desorbed, regenerating the absorbent capacity thereof. The key operating parameter of the desorption column is the operating temperature in the column (90 - 130°C). In the head of the column a gaseous stream of CO₂ is obtained, which passes through a cooling system (16) or condenser in order to recover the water which may have evaporated in the desorption. This stream is mixed with air (17) and is fed by bubbling, before passing through a second compressor (18), to the second reactor (2) or nitrification tank wherein the nitrifying microorganisms can use the CO₂ as a carbon source. Alternatively, the stream of CO₂ and the stream of air may not be mixed, being fed separately to the second reactor (2) or nitrification tank. This second reactor (2) is also fed with nutrients (19) and ammonium necessary for biological activity.

A portion of the liquid stream (22) of alkaline carbonate solution obtained in the bottom of the first reactor (1) or absorption column can be purged towards the second reactor (2) or nitrification tank (as it loses absorbent capacity), and serves as a source of CO₂ dissolved in the liquid in order to feed the nitrifying microorganisms. The purged stream is compensated for in the system by means of feeding fresh carbonate solution from a tank (20) wherein a new reagent solution is prepared using water to do so, water already free from CO₂ (21) coming from the second reactor (2) or nitrification tank. This regenerated liquid stream is fed back to the head of the first reactor (1) or absorption column, closing the cycle of the liquid phase.

## Claims

1. A process for capturing carbon dioxide from inside a gas (3), intended to be carried out in at least two reactors (1,2,13), wherein said process is **characterised in that** it comprises, at least, the steps of:
a.) Introducing an alkaline solution (4) into the first reactor (1) and bubbling or purifying the gas (3) such that the carbon dioxide is absorbed in the form of carbonates in said alkaline solution (4), forming a first liquid (6) comprising solubilised carbonates, and consequently a clean gas stream (5) is formed in an upper portion of the first reactor (1),
b.) feeding the first liquid (6) to the second reactor (2), which in turn comprises chemoautotrophic nitrifying microorganisms (7),
c.) introducing an air stream (9) into the second reactor (2) and a stream of reduced nitrogen nutrients (8), such that the chemoautotrophic microorganisms (7) use the solubilised carbonates of the first liquid (6) to grow, thus generating solid biomass (12) within a resulting liquid (11) in the second reactor (2).

2. The process of claim 1, wherein the gas (3) is a biogas (3') produced from biomass in a previous step comprising anaerobic digestion (15), and wherein said process comprises reusing a portion (12') of the biomass (12) generated in the second reactor (2) in the anaerobic digestion (15) in order to produce the starting biogas.

3. The process of claim 1, which comprises, furthermore, using waste nutrients (8') resulting from the anaerobic digestion (15) and introducing said waste nutrients (8') into the second reactor (2) thus acting as the source of reduced nitrogen nutrients (8).

4. The process of claim 1, comprising adding one or more nutrients (8) reduced into nitrogen comprising, at least one, from among: ammonium, phosphorus, fertilisers, slurries or urea.

5. The process of claim 1, which comprises, furthermore, recirculating the resulting liquid (11) to the first reactor (1) thus acting as an alkaline solution (4).

6. The process of any one of claims 5, comprising adjusting the pH of the resulting liquid (11) recirculated out of the reactor (2) by means of a device (14).

7. The process of claim 1, wherein all the steps are executed at manometric pressures between 0 bar and 6 bar.

8. The process of claim 1, intended to be carried out in three reactors (1,2,13), wherein in step a.) the gas is purified and wherein said process further comprises the step of:
d.) feeding the first liquid (6) comprising solubilised carbonates to a third reactor (13) wherein the carbon dioxide absorbed in step a.) is partially or totally desorbed, regenerating the absorbent capacity thereof, wherein step d.) takes place between steps a.) and b.) and before step c.).

9. The process of claim 8, wherein prior to step d.) the following is carried out:
e.) a step of flashing (14) the first liquid (6) wherein the pressure thereof is reduced.

10. The process of claim 9 comprising:
f.) a step of recirculating a stream rich in CH₄ to the inlet to the first reactor (1), mixing it with the gas (3).

11. The process of any of claims 9 or 10, wherein after step e.) step d.) is carried out.

12. The process of any of claims 8 to 11, wherein the desorption of step d.) takes place at a temperature between 80 and 150°C.

13. The process of any of claims 8 to 12, wherein after step d.) and before step b.) the process comprises:
f.) a cooling step wherein the water evaporated in step d.) is recovered.

14. The process of any of claims 8 to 13, wherein after step c.) the process comprises:
g.) a step of compensating for a stream purged in step c.), and
h.) a step of feeding said current compensated for in step g.) before step a.).

15. The process of any of claims 8 to 14, wherein all the steps are executed at manometric pressures between 2 bar and 15 bar.
